# EUROPÄISCHE PATENTANMELDUNG

(11) **EP 2 036 577 A1**
(43) Veröffentlichungstag der Anmeldung: **18.03.2009**
(21) Anmeldenummer: 07116409.9
(22) Anmeldetag: 14.09.2007
(51) Int. Cl.: A61K 49/00

(54) **Diagnostische Stoffe für die optische bildgebende Untersuchung auf der Basis von nanopartikulären Formulierungen**

(71) Anmelder: mivenion GmbH, 10115 Berlin (DE)
(72) Erfinder: Bahner, Malte, 10435, Berlin (DE)
(74) Vertreter: Vossius & Partner

(57) **Zusammenfassung**

Gegenstand der vorliegenden Erfindung ist die Bereitstellung von nanopartikulären Formulierungen enthaltend ein PEG-alkyl Block-Copolymer und einen nah-infraroten Fluoreszenzfarbstoff, die Herstellung dieser nanopartikulären Formulierungen, pharmazeutisch Zubereitungen enthaltend die erfindungsgemäßen nanopartikulären Formulierungen, sowie deren Anwendung als Kontrastmittel.

## Beschreibung

Gegenstand der vorliegenden Erfindung ist die Bereitstellung von nanopartikulären Formulierungen enthaltend ein PEG-alkyl Block-Copolymer und einen nah-infraroten (NIR) Fluoreszenzfarbstoff, die Herstellung dieser nanopartikulären Formulierungen, pharmazeutische Zubereitungen enthaltend die erfindungsgemäßen nanopartikulären Formulierungen, sowie deren Anwendung als Kontrastmittel.

Der Einsatz bildgebender Verfahren spielt heute bei der Diagnostik, Therapie und Prophylaxe von Erkrankungen eine entscheidende Rolle. In der klinischen Medizin werden jährlich mehr als 300 Millionen bildgebende Untersuchungen durchgeführt. Den Hauptteil dieser Untersuchungen bilden technisch einfach zu handhabende Verfahren wie die Ultraschalltechnik oder die X-ray basierten Untersuchungsverfahren. Eine weiteres, technisch einfach zu handhabendes Verfahren ist die optische Bildgebung.

Die optische Bildgebung ist ein bereits sehr lange etabliertes, bildgebendes diagnostisches Verfahren. Während in ihrer einfachsten Ausführung Weißlicht zur Durchleuchtung von Organen verwendet wird (Diasphanoskopie), kommt bei technisch anspruchsvolleren Verfahren Licht mit genau definierten spektralen Eigenschaften zum Einsatz. Hierdurch können die unspezifischen Hintergrundsignale vermindert werden und gleichzeitig die Eindringtiefe des diagnostischen Lichtes erhöht werden. Beide Parameter beeinflussen maßgeblich die diagnostische Sicherheit der optischen Bildgebung.

Eine weitere Möglichkeit, die Spezifität und die Sensitivität der optischen Bildgebung zu verbessern ist die Verwendung von Fluoreszenzfarbstoffen. Seit etwa drei bis vier Jahrzehnten stehen verschiedene diagnostische Fluoreszenzfarbstoffe zur Verfügung. Einen besonderen Stellenwert hat hierbei die Wirkstoffklasse der Polymethinfarbstoffe, besonders der Cyaninfarbstoffe. Das Indocyaningrün (ICG) ist ein bekannter Fluoreszenzfarbstoff, der eine breite Anwendung in der optischen Bildgebung besitzt. Hauptanwendungsgebiete für das ICG ist die Fluoreszenzangiographie in der Ophthalmologie und in der Gefäßchirurgie. Die Anwendung von ICG ermöglicht die Darstellung der Gefäße. Dadurch ist es dem Arzt z.B. möglich, pathologische Gefäßneubildungen, die auf Krankheiten hinweisen, nachzuweisen. Eine andere, sehr bedeutsame Anwendung besteht in der Gefäßchirurgie. Hier kann durch einen Fluoreszenzfarbstoff geprüft werden, ob bestimmte plastische Eingriffe am Gefäß zu dem erwünschten Erfolg führten. Tritt z.B. ein intravenös injiziertes Kontrastmittel an einer bestimmten Stelle des Gefäßes aus, hat der Chirurg einen sicheren Hinweis, dass der operative Eingriff nicht erfolgreich war. Das ICG ist für diese Anwendungen geeignet, da es nicht das Gefäßbett verlässt und sich nicht im Extravasalraum anreichert, da es vollständig an Plasmaproteine gebunden ist.

Aufgrund der schnellen Elimination von ICG aus der Zirkulation nimmt die diagnostische Signalstärke jedoch schnell ab (Photochem. Photobiol. 2000, 72, 392). Hierdurch ist es dem Arzt nicht möglich, über einen längeren Zeitraum kleinere Gefäße sicher darzustellen. Ein weiterer Nachteil des ICG liegt in seinen schlechten Löslichkeitseigenschaften. Die starke Tendenz der ICG-Moleküle, Molekülaggregate zu bilden, erschwert einerseits die Herstellung einer vollständigen Auflösung der zu verabreichenden Substanzmenge. Ein anderer Nachteil ist die verminderte Fluoreszenzaktivität, die durch aggregierte ICG-Moleküle bewirkt wird. (Microvascular Res. 1998, 55; Survey Opthalmol. 2000, 45, 15). Ein weiterer Nachteil von ICG ist die Instabilität der pharmazeutischen Wirkstofflösung. Der Nachteil des Zerfalls des Wirkstoffs in wässrigen Lösungsmedien wird auch bei anderen Wirkstoffen aus der Klasse der Polymethinfarbstoffe beobachtet. Diese nachteilige Eigenschaft verhindert die Abgabe einer wässrigen Lösung zur Injektion, was mit einem höheren Herstellaufwand verbunden ist.

Dem Fachmann sind verschiedene Wege bekannt, die Nachteile des häufig angewendeten ICG zu beheben. Zum einen ist die Synthese von neuartigen Fluoreszenzfarbstoffen mit verbesserten Eigenschaften bekannt. Die Synthese neuartiger Fluoreszenzfarbstoffe hat zu Wirkstoffen geführt, die verbesserte Löslichkeitseigenschaften aufweisen und eine im Vergleich zu ICG höhere Fluoreszenzquantenausbeute haben. Alle bisher bekannten Stoffe haben jedoch die Tendenz, zu einer bestimmten Zeit nach intravenöser Injektion das Blutgefäßsystem zu verlassen und sich in dem extravasalen Raum anzureichern (Acad. Radiol. 2006, 13, 4; J Fluoresc. 2005, 15, 443). Der Prozess der Extravasation beginnt bereits eine Minute nach intravenöser Injektion. Eine der Ursachen für dieses Verhalten ist eine unvollständige Bindung an Plasmaproteine. Die Extravasation von ICG wird durch die vollständige Bindung von ICG an Plasmaproteine verhindert.

Fluoreszenzfarbstoffe, die die Eigenschaft besitzen, das Gefäßsystem zu verlassen, führen zu einer Erhöhung des Fluoreszenzsignals im extravasalen Gewebe. Hierdurch wird das Signalzu-Hintergrunds-Verhältnis im Vergleich zu ICG schlechter, obwohl die Fluoreszenzquantenausbeute der bekannten Substanzen verbessert wurde.

Eine weitere, dem Fachmann bekannte Methode, neue Fluoreszenzfarbstoffe mit verbesserten Eigenschaften im Vergleich zu ICG darzustellen, ist die Synthese von Farbstoff-Protein-Konjugaten (Technol. Cancer Res. Treat. 2004, 3, 393). Es ist bekannt, dass durch Kopplung an ein Protein mit einem Molekulargewicht von größer 70 kDa oder durch Generierung solcher Molekulargewichte durch Konjugation von Proteinen mit Polyethylenglykolen (Adv. Drug Deliv. Rev. 2003, 55, 1261-77), die renale Ausscheidungsfähigkeit sehr stark vermindert wird. Hierdurch kann unmittelbar eine längere Zirkulationsdauer erreicht werden. Dieses Vorgehen führt aber ebenfalls zu neuen Nachteilen im Vergleich zu ICG. Ein Nachteil ist das erhöhte Risiko einer unerwünschten Wirkung durch die zusätzliche Anwendung von Proteinen. Es ist bekannt, dass Proteine wie Albumine oder Immunglobuline, die als Kopplungspartner verwendet werden, zu einer erhöhten Immunreaktion führen können. Da bei diagnostischen Verfahren eine hohe Arzneistoffsicherheit notwendig ist, ist die Anwendung von Fluoreszenz-Protein-Konjugaten nicht bevorzugt. Ein weiterer Nachteil sind die deutlich höheren Herstellkosten für ein Fluoreszenzfarbstoff-Protein-Konjugat im Vergleich zum ICG.

Die Herstellung veränderter pharmazeutischer Zubereitungen, mit dem Ziel, verbesserte Eigenschaften von ICG zu erreichen, ist ebenfalls bekannt. Verschiedene pharmazeutische Zubereitungen sind beschrieben in Photochem. Photobiol. 2000, 71, 347 (Rajagopalan et al.), WO2007/025768 (Fischer et al.), Polymeric nanoparticulate delivery system for Indocyanine green: Biodistribution in healthy mice. Int. J. Pharm, 2004, 278, 93-301, Saxena, V. et al.: Enhanced photo-stability, thermal stability and aqueous-stability of indocyanine green in polymeric nanoparticulate systems, J. Photochem. Photobiol. B., 2004, 74, 29-38, WO2004/064751.

Es war daher Aufgabe der vorliegenden Erfindung, nanopartikuläre Formulierungen mit verbesserten Eigenschaften zur Verfügung zu stellen. Diese nanopartikulären Formulierungen eignen sich insbesondere als Kontrastmittel.

Gegenstand der vorliegenden Erfindung sind nanopartikulären Formulierungen enthaltend ein PEG-alkyl Block-Copolymer und einen NIR (Nahinfrarot)-Fluoreszenzfarbstoff. PEG-alkyl Block-Copolymere sind polymere amphiphile Substanzen oder Tenside, die in wässrigem Medium definierte Molekülanordnungen, insbesondere Mizellen oder Emulsionen, bilden. Mizellen sind im Sinne der Erfindung Bestandteil nanopartikulärer Formulierungen.

PEG-alkyl Block-Copolymere im Rahmen der Erfindung sind Verbindungen, die Polyethylenglykol (PEG) oder Methoxypolyethylenglykol als hydrophiles Strukturelement und eine Alkylkette als lipophiles Strukturelement enthalten. Erfindungsgemäß sind Polyethylenglykole von 3 bis 150 Oxyethylen-Einheiten (-CH₂CH₂O-), bevorzugt 3 bis 50 Oxyethylen-Einheiten. PEG sind dabei nicht notwendigerweise einheitlich, sondern ein Gemisch bestehend aus einer verschiedenen Anzahl von Oxyethylen-Einheiten mit einem mittleren Molekulargewicht.

Die Alkylkette ist erfindungsgemäß eine Alkylkette aus 3 bis 30 Kohlenstoffatomen, die ein-oder mehrfach unabhängig voneinander mit C1-C3-Alkyl, Hydroxyl oder Phenyl substituiert sein kann. Bevorzugt sind Alkylketten, die sich von gesättigten, ungesättigten oder chemisch/biochemisch modifizierten Fettsäuren ableiten.

Gesättigte Fettsäuren sind beispielsweise Buttersäure, Kapronsäure, Behensäure, Lignocerinsäure, Cerotinsäure, Stearinsäure, Arachinsäure, Margarinsäure, Palmitinsäure, Myristinsäure, Laurinsäure, Kaprinsäure, Kaprylsäure. Ungesättigte Fettsäuren sind beispielsweise Palmitoleinsäure, Vaccensäure, Elaidinsäure, Ölsäure, Icosensäure, Nervonsäure, Erucasäure, Cetoleinsäure, Linolsäure, Linolensäure, Timnodonsäure, Clupanodonsäure, Arachidonsäure. Chemisch oder biochemisch modifizierte Fettsäuren sind Hydroxyfettsäuren oder Aryl-Fettsäuren, beispielsweise 12-Hydroxystearinsäure.

Insbesondere sind daher bevorzugt nanopartikuläre Formulierungen, wobei das PEG-alkyl Block-Copolymer ein PEG-Fettsäureester Block-Copolymer ist.

Insbesondere bevorzugt ist Polyethylenglykol-12-Hydroxystearinsäure.

Erfindungsgemäß bilden Fettsäuren mit der Säuregruppe oder einem Derivat der Säuregruppe eine Bindung zum PEG. Bindungen können Ether, Ester, Amide, Carbamate, Thiocarbamate, Thioether, Harnstoffbindungen sein. Dazu kann die terminale Hydroxylgruppe des PEG in ein Amin, Thiol oder eine andere funktionelle Gruppe überführt worden sein. Bevorzugt sind direkte Bindungen der Säuregruppe der Fettsäure mit dem PEG. Besonders bevorzugt sind dabei Esterbindungen.

Gegenstand der Erfindung sind weiterhin PEG-Fettsäureester Block-Copolymere, bei den Glycerol als Strukturelement kovalent mit dem PEG verbunden ist und die Fettsäure Ester mit den Hydroxylgruppen des Glycerols bildet. Besonders bevorzugt ist Polyethylenglykol-Polyglcerol-Ricinoleat.

NIR-Fluoreszenzfarbstoffe sind Chromophore oder Fluorophore mit nahinfraroter (NIR) Fluoreszenz.

Bevorzugt sind NIR-Fluoreszenzfarbstoffe, die lipophil sind. Lipophile NIR-Fluoreszenzfarbstoffe sind im Sinne der Erfindung solche, die höchstens zwei funktionelle Gruppen ausgewählt aus Sulfonat, Sulfat, Carboxyl, Polyhydroxyalkyl (4 bis 6 Hydroxylgruppen) tragen.

Besonders bevorzugt ist der NIR-Fluoreszenzfarbstoff ausgewählt aus der Gruppe umfassend Polymethinfarbstoffe, Phthalocyanine, Naphthalocyanine, Triphenylmethine, Croconiumfarbstoffe, Squariliumfarbstoffe.

Am meisten bevorzugt ist der NIR-Fluoreszenzfarbstoff ausgewählt aus der Gruppe umfassend Polymethinfarbstoffe, Cyaninfarbstoffe, Indotricarbocyanine, DODCI, DTDCI, DOTCI, DDTCI und Indocyaningrün.

Bevorzugte Indotricarbocyanine basieren auf Benzoindoleninen und einer unsubstituierten oder substituierten Pentamethinkette, sowie jeweils einem mit einer hydrophilen Gruppe substituierten Alkylrest am Stickstoff der Benzoindolstruktur. Insbesondere ist der NIR-Fluoreszenzfarbstoff Indocyaningrün (ICG, CardioGreen, IC Green, DiagnoGreen) bevorzugt. Ganz besonders sind neben dem Indocyaningrün auch Derivate des Indocyaningrün bevorzugt. Derivate des Indocyaningrüns sind die Indocyaningrünstraktur mit einer Polymethinkette, die unabhängig voneinander substituiert sind mit C1-C3-Alkyl, Chlor, C1-C3-Alkyloxy und/oder einem Alkylrest, der zusammen mit der Heptamethinkette des Indocyaningrüns einen 5- oder 6-gliedrigen Ring bildet.

Der Durchmesser der erfindungsgemäßen nanopartikulären Formulierung kann in einem Bereich von 1 nm bis 1000 nm liegen, vorzugsweise 5 nm bis 500 nm, besonders bevorzugt 5 nm bis 50 nm.

Die Fluoreszenz bzw. NIR-Fluoreszenz der erfindungsgemäßen Formulierungen liegen in einem Bereich von 600 nm bis 1000 nm, bevorzugt 750 nm bis 900 nm.

Die Fluoreszenzquantenausbeute der erfindungsgemäßen Formulierungen ist mindestens so hoch wie eine Farbstofflösung in Wasser, bevorzugt jedoch doppelt so hoch, besonders bevorzugt um das Vierfache höher. Für Indocyaningrün ist die Fluoreszenzquantenausbeute in der nanopartikulären Formulierung bevorzugt mindestens 4%, besonders bevorzugt mindestens 8%.

Verglichen mit einer rein wässrigen Formulierung tritt ein Quenching, d. h. eine Abnahme der Fluoreszenzintensität bei weiterem Anstieg der Konzentration, für die erfindungsgemäßen Formulierungen erst bei höheren Konzentrationen auf. Bevorzugt tritt ein Quenching erst oberhalb einer Konzentration von 0,1 mg/mL auf. Für Indocyaningrün tritt ein Quenching bevorzugt bei einer 10-fach höheren Konzentration verglichen mit einer rein wässrigen Lösung auf.

Darüber hinaus lassen sich erfindungsgemäß Formulierungen in höheren Konzentrationen bereitstellen als rein wässrige Formulierungen der Farbstoffe. Bevorzugt ist eine Konzentration von mindestens 0,5 mg/mL, bevorzugt mindestens 1 mg/mL.

Die Lagerstabilität der erfindungsgemäßen Formulierungen ist deutlich verbessert, verglichen mit rein wässrigen Formulierungen und den Formulierungen, die in Saxena et al. beschrieben werden. Sieben Tage nach Herstellung sind noch mehr als 90 % des formulierten NIR-Fluoreszenzfarbstoffes intakt nachweisbar.

Die Plasmaproteinbindung des ICG in den erfindungsgemäßen Formulierungen entspricht der von ICG. Nach Inkubation einer Mizellformulierung in humanem Serum, und Messung des freien Anteils an ICG nach einer Inkubationszeit von 4 h und Abtrennung des ungebundenen ICG mittels Ultrazentrifugation wird ein freier Anteil von < 5% gemessen. Dieser Wert wird im analogen Experiment mit einer wässrigen Lösung von ICG erhalten.

Das Absorptionsmaximum von ICG in der Mizellformulierung ist mit 797 nm im Vergleich mit einer wässrigen Lösung von ICG (Absorptionsmaximum 780 nm) um 17 nm zu höheren Wellenlängen rotverschoben (red shift). Diese Verschiebung führt entsprechend der optischen Eigenschaften von Gewebe zu einer höheren Eindringtiefe des Anregungslichtes und somit zu einer verbesserten Detektion in Geweben. Des Weiteren ist das Absorptionsmaximum bei 797 nm nahe dem Absorptionsmaximum von ICG in Blutplasma (805 nm). Auf diese Wellenlänge sind die etablierten Diagnosegeräte eingestellt, so dass diese Eigenschaft eine direkte Verwendung der Formulierung erlaubt.

Gegenstand der Erfindung ist weiterhin ein pharmazeutisches Mittel enthaltend eine erfindungsgemäße nanopartikuläre Formulierung.

Des Weiteren ist Gegenstand der Erfindung eine erfindungsgemäße nanopartikuläre Formulierung zur Verwendung als Kontrastmittel.

Ebenfalls Gegenstand der Erfindung ist ein Verfahren zur Herstellung einer nanopartikulären Formulierung umfassend die folgenden Schritte:

Die wesentlichen Schritte der Herstellung einer nanopartikulären Formulierung sind (1) Lösen des PEG-Alkyl Block-Copolymer in Wasser, vorzugsweise unter Erhalt einer Konzentration von 0,05 bis 1 g/mL (2) Zugabe des Fluoreszenzfarbstoffes zum Lösungsansatz, so dass eine mizelläre Formulierung entsteht.

### Figurenbeschreibung

Figur 1: Absorptionsspektrum von ICG in Wasser (gestrichelte Linie) und ICG-Mizellen in Wasser nach Herstellung gemäß Beispiel 1 (durchgezogene Linie). Normierung auf Mizellenlösung = 1.
Figur 2: Fluoreszenzemissionsspektrum von ICG in Wasser (gestrichelte Linie) und ICG-Mizellen in Wasser nach Herstellung gemäß Beispiel 1 (durchgezogene Linie). Normierung auf Mizellenlösung = 1.
Figur 3: Fluoreszenzintensität von ICG in Wasser (Quadrate) und der ICG-Mizellen in Wasser in Abhängigkeit der Konzentration von ICG (0.001 mg/mL bis 5 mg/mL).
Figur 4: Partikelgrößenverteilung mittels dynamischer Lichtstreuung.
Figur 5: Bestimmung der Stabilität durch Messung der Absorption im Maximum der jeweiligen Formulierung in Abhängigkeit von der Zeit. Vergleich von ICG in Wasser (Quadrate) mit drei ICG-Mizellformulierungen; Solutol 10-proz. (Kreise), 20-proz. (Dreiecke), 40-proz. (Dreiecke auf Spitze stehend).

Weiterhin wird die Erfindung durch die nachfolgenden Beispiele erläutert:

### Beispiele

### Herstellung der Mizellformulierung

Beispiel 1: 2 g Solutol HS 15 werden auf 65°C erhitzt. Unter Rühren werden 10 ml Wasser für Injektionszwecke dazu gegeben und die klare Lösung auf Raumtemperatur abgekühlt. 50 mg ICG werden in der Mizell-Lösung gelöst und durch einen 0,2 µm Membranfilter steril filtriert.

Beispiel 2: 2 g Solutol HS 15 werden bei Raumtemperatur unter Rühren in 10 ml Wasser für Injektionszwecke gegeben. Es entsteht eine klare Lösung. 50 mg ICG werden in der Mizell-Lösung gelöst und durch einen 0,2 µm Membranfilter sterilfiltriert.

### Absorptions- / Fluoreszenzmessungen

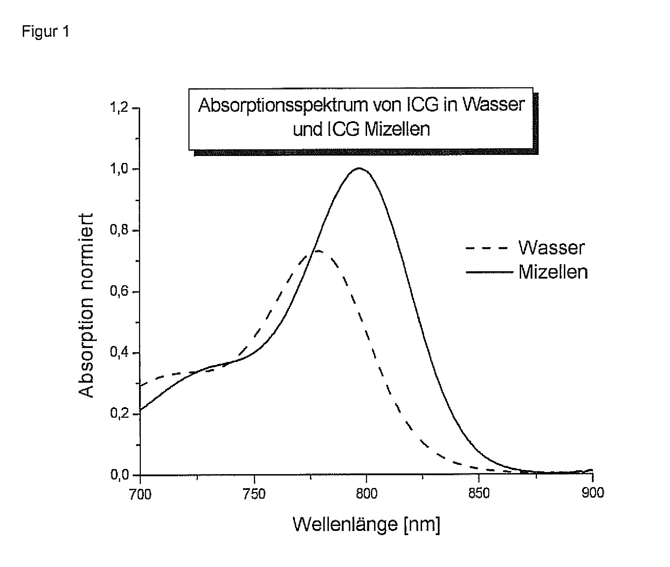

Absorptionsspektren in einem Wellenlängenbereich von 700 nm bis 900 nm wurden mit einem UVIKON 933 Spectrophotometer (Firma Kontron) gegen das jeweilige Lösungsmittel aufgenommen.
→ ICG in Wasser λmax = 780 nm
   ICG-Mizellen λmax = 797 nm (Figur 1)

Die Fluoreszenzmessungen wurden an einem FluoroLog-2 Spectrofluorometer (350 W Xenon Lampe) der Firma Spex durchgeführt. Dazu wurden Emissionsspektren von 700 nm bis 900 nm aufgenommen. Die Exzitationswellenlänge entsprach dem jeweiligen Maximum der Formulierung im Absorptionsspektrum (ICG in Wasser λmax = 780 nm und ICG-Mizellen λmax = 795 nm). Durch den s & r Modus der Software DM 3000 konnten bei der Auswertung die unterschiedlichen Lampenintensitäten der verschiedenen Anregungswellenlängen verrechnet werden.

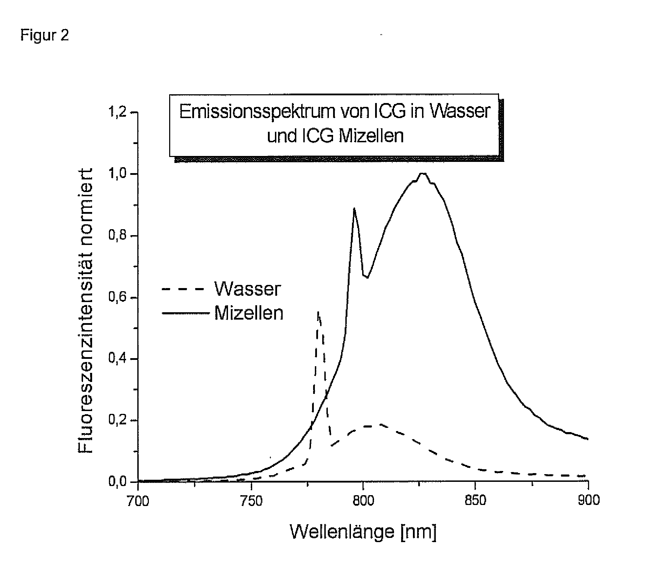

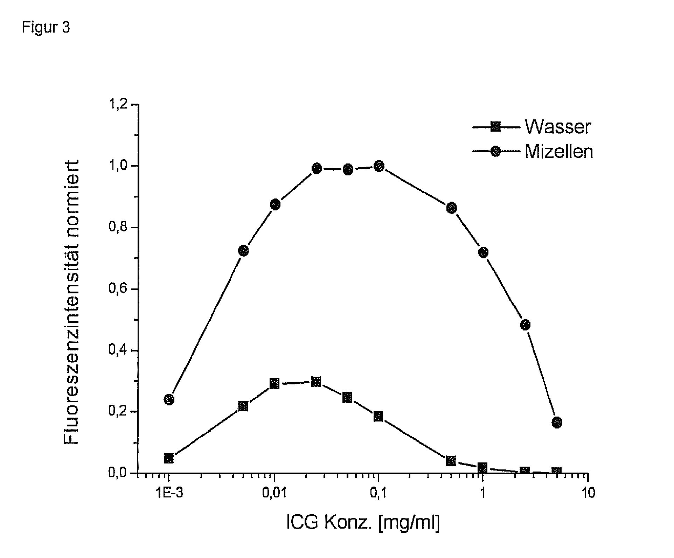

Die Quantenausbeute berechnet sich über die Fläche unter der Emissionskurve. Als Standard wurde ICG in DMSO verwendet (Φ = 0,13).
→ Die Quantenausbeute von ICG-Mizellen beträgt Φ = 0,08 im Vergleich zu ICG in Wasser Φ = 0,02. Ein Quenching tritt erst bei höheren Konzentrationen (ab 0,1 mg/ml ICG) auf (Figur 2 und Figur 3).

### Stabilitätsuntersuchungen

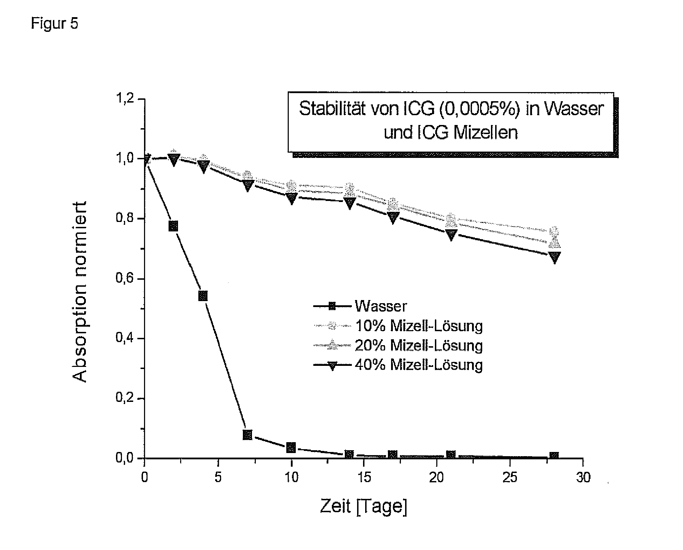

Für den Stabilitätstest wurde die Absorption im Maximum der jeweiligen Formulierung in Abhängigkeit von der Zeit gemessen. Dazu wurden 0,0005%ige ICG-Lösungen hergestellt und bei Raumtemperatur gelagert. Die Lagerung der rein wässrigen ICG-Formulierung zeigt eine Reduktion der normierten Absorption auf unter 10% bereits nach 7 Tagen. Dagegen zeigen die Mizell-Formulierungen von ICG nach 7 Tagen Lagerung noch mehr als 90% der Absorption im Vergleich zum Startwert, die auch nach 4 Wochen nicht unter 70% sinkt (Figur 5).

### Partikelgröße

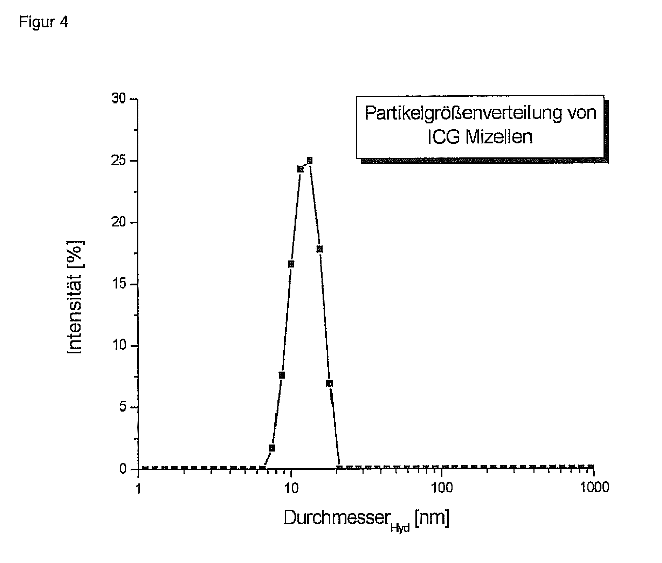

Die Partikelgrößenverteilung wurde mittels dynamischer Lichtstreuung (Zetasizer NS, Firma Malvern) bestimmt. Gemessen wurde mit einem He-Ne-Laser (633 nm, 4 mW) aus einem Winkel von 173°. Die Proben wurden direkt ohne Verdünnung in 45 µl Quarzküvetten vermessen.
→ ICG-Mizellen haben einen hydrodynamischen Durchmesser von 12 nm bei einem PDI (Polydispersitätsindex) von 0,061. (Figur 4)

### Plasmaproteinbindung

Zur Bestimmung der Plasmaproteinbindung wurde der Wellenlängenshift im Absorptionsspektrum beobachtet. Dazu wurden Spektren von 700 nm bis 900 nm von ICG in Wasser und in Plasma verglichen mit Spektren von ICG-Mizellen in Wasser und in Plasma.
→ Bei beiden Formulierungen verschiebt sich das Absorptionsmaxium in Plasma zu 805 nm. Das Verhalten der Plasmaproteinbindung des ICG in den erfindungsgemäßen Formulierungen entspricht der von ICG in wässrigem Medium.

### Hämolyse-Assay

Für die Untersuchung der hämolytische Aktivität von ICG-Mizellen wurde heparinisiertes humanes Vollblut zunächst vom Plasma entfernt und 3x mit PBS Puffer gewaschen. Nach Herstellung einer 2%igen Erythrozytensuspension in PBS wurde diese mit der ICG-MizellFormulierung für 1 h bei 37°C inkubiert. Als Blindwert (0% Hämolyse) diente reine PBS Lösung und als 100% Hämolysewert 2%ige Triton Lösung. Nach Inkubation wurden die Erythrozyten abzentrifugiert und die Rotfärbung im Überstand photometrisch bei 540 nm bestimmt.
→ ICG-Mizellen zeigen keine hämolytische Aktivität.

## Patentansprüche

1. Nanopartikuläre Formulierung enthaltend ein PEG-alkyl Block-Copolymer und einen nah-infraroten Fluoreszenzfarbstoff.

2. Nanopartikuläre Formulierung gemäß Anspruch 1, wobei das PEG-alkyl Block-Copolymer ein PEG-Fettsäureester Block-Copolymer ist.

3. Nanopartikuläre Formulierung gemäß einem der Ansprüche 1 oder 2, wobei der nahinfrarote Fluoreszenzfarbstoff ein lipophiler Fluoreszenzfarbstoff ist.

4. Nanopartikuläre Formulierung gemäß einem der Ansprüche 1 bis 3, wobei der nahinfrarote Fluoreszenzfarbstoff ausgewählt ist aus der Gruppe umfassend Polymethinfarbstoffe, Phthalocyanine, Naphthalocyanine, Triphenylmethine, Croconiumfarbstoffe, Squariliumfarbstoffe

5. Nanopartikuläre Formulierung gemäß einem der Ansprüche 1 bis 4, wobei der nahinfrarote Fluoreszenzfarbstoff ausgewählt ist aus der Gruppe umfassend Polymethinfärbstoffe, Cyaninfarbstoffe, Indotricarbocyanine, DODCI, DTDCI, DOTCI, DDTCI, Indocyaningrün und Derivate des Indocyaningrüns.

6. Nanopartikuläre Formulierung gemäß einem der Ansprüche 1 bis 5, wobei der NIR-Fluoreszenzfarbstoff Indocyaningrün ist.

7. Phannazeutisches Mittel enthaltend eine nanopartikuläre Formulierung gemäß einem der Ansprüche 1 bis 6.

8. Nanopartikuläre Formulierung gemäß einem der Ansprüche 1 bis 6 zur Verwendung als Kontrastmittel.

9. Verfahren zur Herstellung einer nanopartikulären Formulierung gemäß einem der Ansprüche 1 bis 6 umfassend die folgenden Schritte: (1) Lösen des PEG-Alkyl Block-Copolymer in Wasser, (2) Zugabe des Fluoreszenzfarbstoffes zum Lösungsansatz, sodass eine mizelläre Formulierung entsteht.
